# EUROPEAN PATENT SPECIFICATION

(11) **EP 3 064 203 B1**
(45) Date of publication and mention of the grant of the patent: **26.12.2018**
(21) Application number: 14858158.0
(22) Date of filing: 23.10.2014
(51) Int. Cl.: A61K 31/08, A61P 1/16, A61P 1/00, A23L 2/52, A23L 33/12

(54) **PHARMACEUTICAL COMPOSITION FOR PREVENTING OR TREATING NONALCOHOLIC STEATOHEPATITIS, CONTAINING BATYL ALCOHOL AS EFFECTIVE COMPONENT**
PHARMAZEUTISCHE ZUSAMMENSETZUNG ZUR PRÄVENTION ODER BEHANDLUNG VON NICHTALKOHOLISCHBEDINGTE STEATOHEPATITIS MIT BATYLALKOHOL ALS WIRKSTOFF
COMPOSITION PHARMACEUTIQUE SERVANT À LA PRÉVENTION OU AU TRAITEMENT DE LA STÉATOHÉPATITE NON ALCOOLIQUE CONTENANT COMME CONSTITUANT EFFICACE DE L'ALCOOL BATYLIQUE

(30) Priority: 29.10.2013 KR 20130129213; 22.10.2014 KR 20140143563
(43) Date of publication of application: 07.09.2016
(73) Proprietor: University Of Ulsan Foundation For Industry Cooperation, Ulsan 680-749 (KR)
(72) Inventor: LEE, Ki Up, Yangpyeong-gun Gyeonggi-do 476-821 (KR); KOH, Eun Hee, Seoul 138-930 (KR); JANG, Jung Eun, Seoul 138-873 (KR)
(74) Representative: Roos, Peter
(86) International application number: PCT/KR2014/010030
(87) International publication number: WO 2015/064960

(56) References cited:
- KR-A- 20060 006 973
- US-A1- 2012 082 719
- US-B1- 6 252 060
- MINGSHUN ZHANG ET AL: "Oral Administration of Alkylglycerols Differentially Modulates High-Fat Diet-Induced Obesity and Insulin Resistance in Mice", EVIDENCE-BASED COMPLEMENTARY AND ALTERNATIVE MEDICINE : ECAM, vol. 2013, no. 11, 1 January 2013 (2013-01-01), pages 1-11, XP055371315, United States ISSN: 1741-427X, DOI: 10.1155/2013/834027
- ELISA FABBRINI ET AL: "Obesity and nonalcoholic fatty liver disease: Biochemical, metabolic, and clinical implications", HEPATOLOGY, vol. 51, no. 2, 1 February 2010 (2010-02-01), pages 679-689, XP055371833, ISSN: 0270-9139, DOI: 10.1002/hep.23280
- BRAVERMAN, N. E. ET AL.: 'Functions of plasmalogen lipids in health and disease' BIOCHIMICA ET BIOPHYSICA ACTA vol. 1822, no. 9, 2012, pages 1442 - 1452, XP028432535
- TESSARI, P. ET AL.: 'Hepatic lipid metabolism and non-alcoholic fatty liver disease' NUTRITION, METABOLISM AND CARDIOVASCULAR DISEASES vol. 19, no. 4, 2009, pages 291 - 302, XP026092333

## Description

### TECHNICAL FIELD

The present invention relates to a pharmaceutical composition and a health food composition that are each for preventing or treating nonalcoholic steatohepatitis by including the plasmalogen precursor batyl alcohol.

### BACKGROUND ART

Liver is the organ that plays a key role in nutrient metabolism, weighing about 1,500 g in human subjects. Abnormal liver function may cause problems in the nutrient metabolism, including defects in glycogen synthesis from glucose, converting protein into albumin, or decomposing and delivering unnecessary materials to be excreted through bile.

The most common cause of liver disease is hepatitis, and this can be classified into acute and chronic, and also into viral hepatitis, alcoholic hepatitis, or drug-induced hepatitis depending on the causes. A spectrum of liver disease also includes hepatic steatosis, hepatitis (steatohepatitis), liver cirrhosis, and liver cancer. Mechanisms of progression of the liver diseases have not been completely established, but hepatic steatosis is one of the primary lesions that lead to secondary hepatocyte damage. Thus effective control of hepatic steatosis may prevent occurrence of severe liver diseases. Currently, the liver diseases have been treated by exercise, abstention from alcohol, or dietary therapy, accompanied by drug treatment. However, there has been no treatment method that fundamentally or completely treats liver diseases.

Liver pills prescribed on the market have prevention and treatment effects with respect to acute liver damages in animals or clinical trials in many cases, and the pills include silymarin (e.g., Legalon®), UDCA (e.g., Urusa®), or PMC (Nissel™). However, the pills or any other agents have not yet exhibited significant treatment effects.

Recently, peroxisome, an intracellular organelle, as well as mitochondria have been shown to play an important role in fatty acid oxidation. Abnormal peroxisome function has been reported to cause hepatic steatosis. Also, peroxisome decomposes reactive oxygen species and is essential for synthesis of a material known as plasmalogen.

Plasmalogen belongs to phospholipids that has a vinyl ether bond, and is a main component of a cell layer. Plasmalogen is also known to play an important role in maintaining cell function by acting as a signaling molecule or producing ant-oxidation effects.

Korean Patent No. 10-2011-7015617 discloses that a pharmaceutical composition including a plasmalogen compound is used to treat or prevent membrane cholesterol-related aging diseases such as neurodegeneration, cognitive impairment, osteoporosis, bipolar disorder, and vascular diseases. However, treatment or prevention of liver diseases by using plasmalogen as disclosed in the present invention have not been described.

MINGSHUN ZHANG ET AL., "Oral administration of Alkylglycerols Differentially modulates High-Fat-Diet-Induced Obesity and Insulin Resistance in Mice", EVIDENCE-BASED AND ALTERNATIVE MEDICINE: ECAM, vol.2013(11),1-11 discloses that shark liver oil which comprises batyl alcohol and selachyl alcohol has the potency to decrease HF-induced obesity due to the selachyl alcohol. Batyl alcohol had no effect.

In this regard, the present inventors have discovered that the plasmalogen precursor batyl alcohol suppresses accumulation of fats in the liver and expression of inflammatory genes. We thus completed the present invention to provide a pharmaceutical composition and a health food composition for preventing or treating nonalcoholic steatohepatitis, wherein each of the compositions includes the plasmalogen precursor batyl alcohol.

### DETAILED DESCRIPTION OF THE INVENTIVE CONCEPT

### TECHNICAL PROBLEM

The present invention provides a pharmaceutical composition and a health food composition, which prevent or treat nonalcoholic steatohepatitis by decreasing fat accumulation in the liver and suppressing expression of genes that causes inflammation in liver tissues, and each of the compositions includes the plasmalogen precursor batyl alcohol.

### TECHNICAL SOLUTION

The present invention is a pharmaceutical composition and a health food composition, which prevent or treat nonalcoholic steatohepatitis by decreasing accumulation of fats in liver and suppressing expression of genes that causes inflammation in liver tissues. This includes the plasmalogen precursor batyl alcohol.

The plasmalogen precursor is an alkoxy glycerol having a C18 fatty alcohol linked to a glycerol backbone via an ether bond. In particular, the alkoxy glycerol is batyl alcohol.

Batyl alcohol decreases accumulation of neutral fats, and suppresses expression of inflammatory cytokine genes, and increases fatty acids oxidation of peroxisome.

The liver disease is nonalcoholic steatohepatitis.

The invention may contain batyl alcohol at an amount of 0.01 part to about 90 parts (by weight) of the 100 parts (by weight) of the pharmaceutical agent.

The invention can be provided as a health food composition that includes batyl alcohol as an effective component.

### ADVANTAGEOUS EFFECTS

According to the present invention, batyl alcohol, can prevent or treat nonalcoholic steatohepatitis by decreasing accumulation of neutral fats in the liver, suppressing expressions of inflammatory cytokine genes, and increasing fatty acid oxidation capacity in peroxisome.

### DESCRIPTION OF THE DRAWINGS

Figure. 1 is a graph showing changes in plasma alanine aminotransferase (ALT) level in a group of experimental mice treated with methionine-choline deficient diet (MCDD), which is an animal model of human nonalcoholic steatohepatitis. A group of experimental mice was treated with MCDD and 100 mg/kg/day of plasmalogen precursor alkoxy glycerol. P*** < 0.001 vs a control group (CON); P# < 0.05 vs MCDD;
Figure. 2 is a graph showing changes in neutral fat amounts in livers of the group of experimental mice treated with MCDD and the group of experimental mice treated with MCDD and 100 mg/kg/day of plasmalogen precursor alkoxy glycerol. P* < 0.05 vs CON; P# < 0.05 vs MCDD;
Figure. 3 is a graph showing changes in the expression of inflammatory cytokine genes of the group of experimental mice treated with MCDD and the group of experimental mice treated with MCDD and 100 mg/kg/day of plasmalogen precursor alkoxy glycerol. P* < 0.05 vs CON; P# < 0.05 vs MCDD;
Figure. 4 shows the results of histological change in livers of the group of experimental mice treated with MCDD and the group of experimental mice treated with MCDD and 100 mg/kg/day of plasmalogen precursor alkoxy glycerol;
Figure. 5 is a graph showing mitochondrial fatty acid oxidation (left) and peroxisome fatty acid oxidation (right) as the results of changes in liver tissue fatty acid oxidation capacity of the group of experimental mice treated with MCDD and the group of experimental mice treated with MCDD and 100 mg/kg/day of plasmalogen precursor alkoxy glycerol. P* < 0.05 vs CON; P# < 0.05 vs MCDD; and
Figure. 6 is a graph that shows the results of live histological change and confirms levels of liver neutral fats and plasma ALT of the group of experimental mice treated with HFD and a group (AG-200) of experimental mice treated with HFD and 200 mg/kg/day of plasmalogen precursor alkoxy glycerol.

### BEST MODE

The present invention may provide a pharmaceutical composition for preventing or treating nonalcoholic steatohepatitis and the composition includes the plasmalogen precursor batyl alcohol as an effective component.

The plasmalogen precursor is an alkoxy glycerol that has C18 fatty alcohol linked to a glycerol backbone via an ether bond. In particular, the alkoxy glycerol is batyl alcohol.

In one embodiment, batyl alcohol may be administered orally.

The plasmalogen precursor, may decrease accumulation of neutral fats, suppress expression of inflammatory cytokine genes, and increases fatty acid oxidation of peroxisome.

The liver disease is nonalcoholic steatohepatitis.

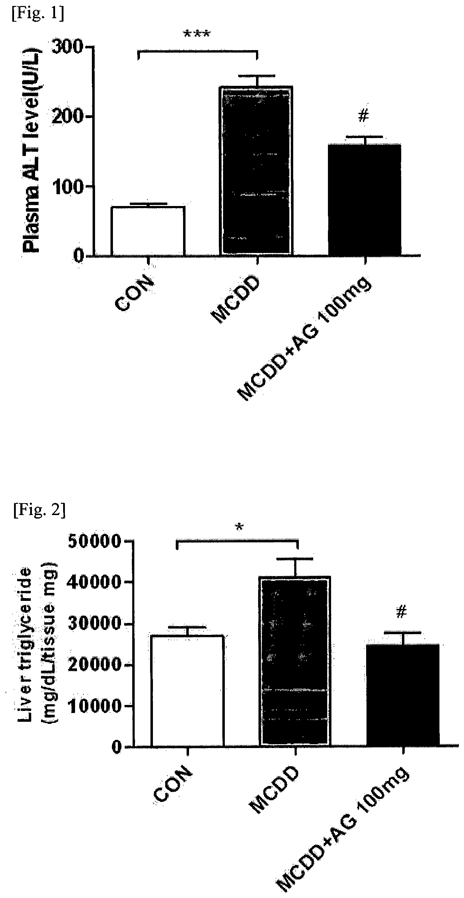

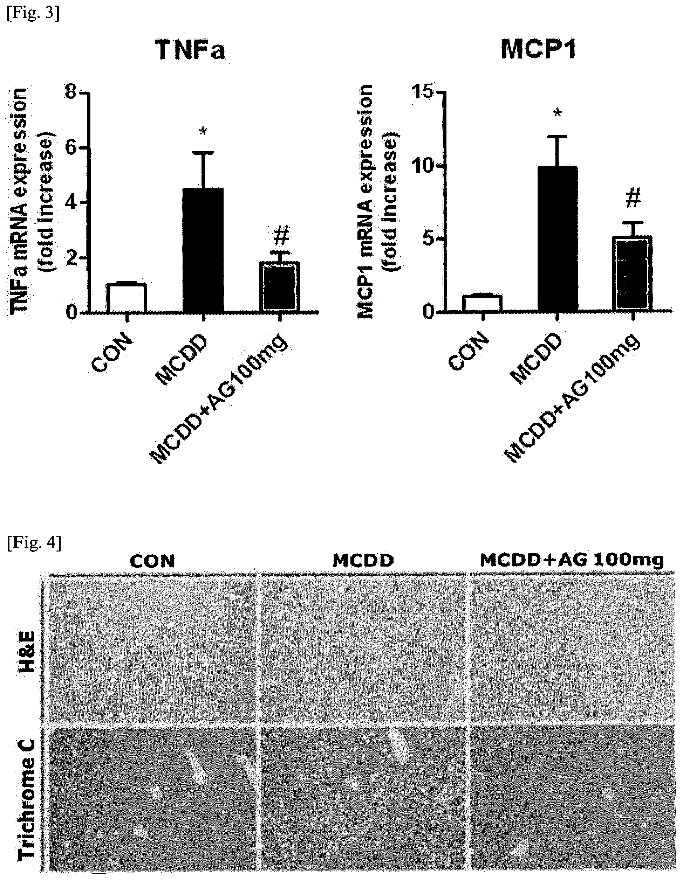

For example, plasma levels of alanine aminotransferase (ALT) were significantly decreased in the animal group given diet that induces hepatic steatohepatitis (i.e., MCDD) and together with alkoxy glycerol, which is a plasmalogen precursor, than the control group given MCDD only (Figure 1). Real-time gene amplification of the liver tissues, confirmed that expression of inflammatory cytokine genes was suppressed in MCDD + alkoxyl glycerol group as shown in Fig. 3. In this regard, the plasmalogen or an analog thereof produced positive effects in terms of preventing or treating hepatitis and liver cirrhosis.

Also, as a result of confirming neutral fats in the liver by measuring triglyceride, the experimental group administered with the MCDD together with alkoxy glycerol, as a plasmalogen precursor, showed improvement in hepatic steatosis as comparable to the results of a control group administered with normal diet as shown in Fig. 2. In addition, as shown in Fig. 4, the experimental group showed significant improvement in the liver fibrosis. In this regard, it can be concluded that the plasmalogen precursor produces excellent effects in terms of preventing or treating a hepatic steatosis, stetohepatitis and fibrosis.

The plasmalogen precursor may be contained at an amount in a range of about 0.01 part to about 90 parts by weight based on 100 parts by weight of the total weight of the pharmaceutical composition.

In one embodiment of the present invention, a pharmaceutical composition for preventing or treating nonalcoholic steatohepatitis, the composition including batyl alcohol as an effective component, may be used in any formulation selected from the group consisting of injections, granules, powders, tablets, pills, capsules, suppositories, gels, suspensions, emulsions, drops, or solutions.

In another embodiment of the present invention, a pharmaceutical composition for preventing or treating nonalcoholic steatohepatitis, the composition including the plasmalogen precursor, as an effective component, may include at least one additive selected from the group consisting of appropriate carriers, excipients, disintegrating agents, sweetening agents, coating agents, swelling agents, glidants, flavors, antioxidants, buffers, bacteriostatic agents, diluents, dispersing agents, surfactants, additives, and lubricants.

In particular, the carriers, excipients, and diluents may be prepared by using lactose, dextrose, sucrose, sorbitol, mannitol, xylitol, erythritol, maltitol, starch, acacia rubber, alginate, gelatin, calcium phosphate, calcium silicate, cellulose, methyl cellulose, microcrystalline cellulose, polyvinylpyrrolidone, water, methyl hydroxybenzoate, propyl hydroxybenzoate, talc, magnesium stearate, or mineral oil. Examples of a solid formulation for oral administration may include tablets, pills, powders, granules, and capsules, and the solid formulation may be prepared by mixing at least one excipient, for example, starch, calcium carbonate, sucrose or lactose, or gelatin to the composition. Also, lubricants such as magnesium stearate or talc, in addition to a simple excipient, may be used. Examples of the solution formulation for oral administration may include suspensions, liquids for internal use, emulsions, or syrups, and various excipients, such as wetting agents, sweetening agents, aromas, and preservatives, may be included in addition to water or liquid paraffin, which is a simple diluent that is commonly used. Examples of a formulation for parenteral administration may include sterilized aqueous solutions, non-aqueous solvents, suspending agents, emulsions, lyophilized preparations, or suppositories. Examples of the non-aqueous solvents or suspending agents may include propylene glycol, polyethylene glycol, vegetable oil such as olive oil, or injectable esters such as ethyloleate. Materials for the suppositories may include witepsol, macrogol, tween 61, cacao paper, laurin paper, glycerogelatin.

According to one embodiment of the present invention, the pharmaceutical composition may be administered to the subject by using a common method via intravenous, intra-arterial, intraperitoneal, intramuscular, intrasternal, percutaneous, endonasal, inhalation, topical, rectal, oral, intraocular, or intradermal routes.

A preferable dose of batyl alcohol may differ depending on the condition and weight of the subject, types and degrees of the disease, form of drugs, and routes and periods of the administration, and the dose may be appropriately selected by one of ordinary skill in the art. According to one embodiment of the present invention, the dose may be in a range of, but not limited to, about 0.01 mg/kg to about 200 mg/kg per day, or, for example, about 0.1 mg/kg to about 200 mg/kg per day, or about 0.1 mg/kg to about 100 mg/kg per day. The administration may be performed once a day or several times in a day, but embodiments are not limited thereto.

In the present invention, the 'subject' may be a mammal including a human, but is not limited thereto.

According to another embodiment, provided may be a health food composition for improving or preventing nonalcoholic steatohepatitis, and the composition includes batyl alcohol effective component.

The health food may be provided in the form of powders, granules, tablets, capsules, syrups, or drinks, and the health food may be used together with food other than the plasmalogen precursor, as an effective component according to the present invention or food additives and may be appropriately used according to a common method. A mixture amount of the effective component may be appropriately determined depending on the purpose of use, for example, for prevention, health, or remedial treatment.

An effective dose of the plasmalogen precursor, included in the health food may be determined based on an effective dose of the pharmaceutical composition, but in the case of long term administration for the purpose of health or hygiene or to control health, the effective dose may be within this range of less. Also, the effective component has no problem in terms of safety, and thus it is certain that the effective dose may be used within this range or more.

A type of the health food is not particularly limited, and examples thereof may include meats, sausages, breads, chocolates, candies, snacks, cookies, pizza, Ramen or other noodles, gums, dairy products including ice cream, soups, beverages, teas, drinks, alcohol drinks, and vitamin complexes.

### MODE OF THE INVENTIVE CONCEPT

Hereinafter, the present invention will be described in detail with reference to the following examples. However, the examples are only for the purpose of illustrations only, not intended to limit the scope of the invention. It should be understood that the examples are provided for a more definite explanation to an ordinary person skilled in the art.

The following Reference Example is described to provide a reference example that commonly applies to each of embodiments according to the present invention.

### <Reference Example>

### 1. Experiment animal and reagent

A methionine-choline deficient (MCD) feed to induce steatohepatitis was purchased from Dyets (Bethlehem, PA, USA). Alkoxy glycerol (batyl alcohol), as a plasmalogen precursor, was purchased from Sigma (St Louis, MO, USA).

2% BSA was prepared in a DMEM:F12 medium, and plasmalogen precursor alkoxy glycerol dissolved in ethanol was added thereto After incubation at 70°C for 20 minutes, the mixture was inoculated in a manner of observing crystal disappearance. 7-week-old C57/BL6 male mice were brought in to an experimental animal room, stabilized for 7 days, and then separated into a general feed group, as a control group, an MCD diet group (an MCDD group), and a group taken a mixture of MCD diet and 100 mg/kg/day of plasmalogen precursor alkoxy glycerol (an AG group), where each of the groups had six mice. The test feeds were taken for the total of 6 weeks, and the mice were allowed to only take water for the following 12 hours. Then, blood samples were obtained from the mice, and liver tissues were harvested from each of the mice. The harvested liver tissues were instantly rapid-frozen in dry ice and stored with plasma in a freezer at -70°C.

### <Example 1> Blood sample measurement

Blood samples were obtained from venae cavae of the experimental animals, the plasma was separated from the blood samples by using a centrifuge (at 300 rpm for 15 minutes), and alanine aminotransferase (ALT) of the plasma was measured by using a kit of Labs Biotechnology (London, Canada). The ALT is a marker that sensitively reflects degrees of inflammation and damage on liver cells, which is widely used to figure out a status of various liver diseases such as hepatitis or liver cirrhosis, as well as hepatic steatosis. In regard of the change in plasma ALT per experimental group, the AG group, which was administered with MCD diet together with 100 mg/kg/day of plasmalogen precursor alkoxy glycerol, showed significantly improvement compared to that of the MCDD group as shown in Fig. 1.

### <Example 2> Neutral fats in liver tissue measurement

Neutral fats in the liver tissue was measured by using a triglyceride (GPO-Trinder) kit purchased from Sigma. The titrated liver tissue and plasma were allowed to react with an extraction buffer at room temperature for 4 hours, IN H₂SO₄ was added thereto, centrifuged at 100 rpm for 10 minutes, and 100 mg of Na₂S₂O₂ was added to a remaining solution after removing a supernatant the centrifuged resultant, and the resultant was centrifuged at 1000 rpm for 5 minutes. Then, a supernatant was obtained therefrom, 0.5 g of silicic acid was added thereto, allowed to react at room temperature for 5 minutes, centrifuged at 1000 rpm for 10 minutes, the supernatant was evaporated with N₂ gas to obtain a sample, and the sample was dissolved with isopropanol. Then, a triglyceride reagent A was added thereto, allowed to react at room temperature for 5 minutes, and the measurement was performed by using an Emax precision micro plate reader (Molecular Devices, Sunnyvale, CA) at 540 nm. A change in an amount of neutral fats of liver tissues per experimental group is the same as shown in Fig. 2, as resulted in regard of the plasma ALT, the AG group, which was administered with MCD diet together with 100 mg/kg/day of plasmalogen precursor alkoxy glycerol, showed significantly improvement results.

### <Example 3> Inflammation change of liver tissue

In order to confirm change in an inflammation degree of liver tissues, gene expression of a tumor necrosis factor (TNF-α) and monocyte chemotactic protein-1 (MCP-1), which are typical inflammatory cytokines, were confirmed by real-time PCR. As a result, referring to Fig. 3, as resulted in regard of the plasma ALT and the amounts of neutral fats in the liver, it was confirmed that administration of 100 mg/kg/day of plasmalogen precursor alkoxy glycerol suppressed expression of inflammatory cytokine genes that was increased by MCD diet.

### <Example 4> Histological observation

A part of the harvested liver tissues were fixed with 10% neutral formalin solution, and embedded in paraffin, and formaldehyde was washed in running water for 12 hours or more. Then, the resultant was step-wisely washed with 60% ethanol for 1 hour, 70% ethanol for 1 hour, 80% ethanol for 1 hour, 90% ethanol for 1 hour, 5% ethanol for 1 hour, and 100% ethanol for 1 hour. Thereafter, the total of three transparent processes and two penetration processes were each performed for 1 hour in xylene, and the resultant underwent an embedding process to prepare a segment having a thickness of about 4 µm.

The segment was died with hematoxylin-eosin (H&E) and trichrome C, and examined and analyzed by using an optical microscope.

Representative H&E and trichrome C dye images of the control group, MCDD group, and AG group are as shown in Fig. 4, and it may be observed that steatohepatitis was relieved to the similar level with that of the control group by the administration of 100 mg/kg/day of plasmalogen precursor alkoxy glycerol.

### <Example 5> Fatty acid oxidation measurement

In order to measure fatty acid oxidation in the liver tissue, ¹⁴CO₂ production amounts from ¹⁴C-palmitate (NEN Life Sciences, Boston, MA, USA) was measured and analyzed.

In this regard, a reaction buffer including 0.2 mM of palmitate (1-¹⁴C-palmitate at 0.5 µCi/ml) was added to 50 µl of a liver titrate, and this was allowed to react at 30°C for 2 hours.

Then, 50 µl of 4 N sulfuric acid, as a stopping buffer, was added thereto, and the mixture was allowed to react for 2 hours, and then a ¹⁴CO₂ production amount was measured. In order to examine fatty acid oxidation of peroxisome, which is in charge of fatty acid beta oxidation, other than mitochondria, 12.5 µM of rotenone, as a mitochondria suppressor, and 100 µM of antimycin were added to a reaction buffer together with 0.2 mM of palmitate (1-¹⁴C palmitate at 0.5 µCi/ml), and the reaction mixture was allowed to react at 30°C for 2 hours. Then, in the same manner, 50 µl of 4 N sulfuric acid, as a stopping buffer, was added thereto, allowed to react for 2 hours, and then a ¹⁴CO₂ production amount was measured.

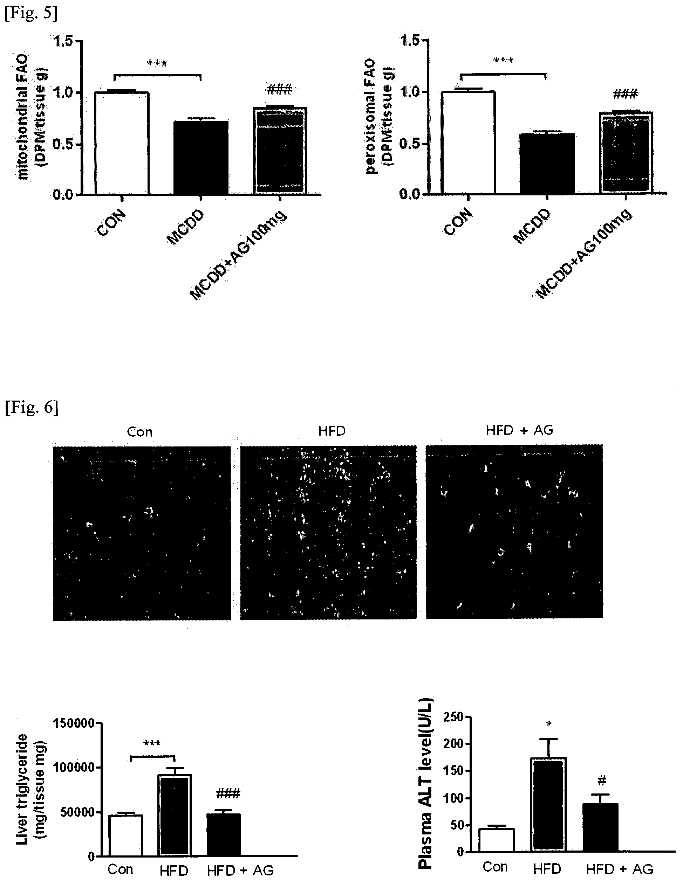

As the result, fatty acid oxidation of peroxisome in the liver tissues per experimental group was confirmed as shown in Fig. 5.

### <Example 6> Confirmation of plasmalogen precursor effect in high fat diet hepatic steatosis animal model

Although a MCDD animal model is a model that represents nonalcoholic steatohepatitis occurrence, the MCDD animal model cannot represent a risk factor that accompanies metabolic syndrome. In order to overcome such limitation, liver tissues of mice that were allowed to take high fat diet (HFD) as a general animal model with respect to hepatic steatosis were used for the comparison.

The animal model only had simple hepatic steatosis without inflammation unlike the MCDD group.

Alkoxy glycerol, as a plasmalogen precursor, was added to the HFD feed that induces obesity and non-alcoholic hepatic steatosis, and the feed was administrated to the animal model, and the change was confirmed.

First, the control group (CON) was prepared by allowing 7-week-old C57/BL6 male mice to take a general feed including 7% of fat, 18% of protein, and 75% of carbohydrate, and the HFD group was prepared by allowing 7-week-old C57/BL6 male mice to freely take a HFD feed including 60% of fat, 18% of protein, and 22% of carbohydrate.

Also, the AG group (AG-200) was allowed to take the HFD mixed with 200 mg/kg/day of alkoxy glycerol. The experimental groups were allowed to take the corresponding feeds for the total of 12 weeks, and liver histological changes in the livers were confirmed in the same manner in Example 4, and the plasma ALT levels were confirmed in the same manner as in Example 1.

As the result, as shown in Fig. 6, it was confirmed that the AG group (AG-200) administered with the HFD together with alkoxy glycerol showed significant improvement in terms of hepatic steatosis occurrence and lipid content in the liver tissues and remarkable reduction in the plasma ALT level, which is a liver damage marker.

Hereinafter, formulation examples of the pharmaceutical composition of the present invention will be described, but the examples are for the purpose of illustrations only, not intended to limit the scope of the invention.

### <Formulation Example 1> Preparation of injection

10 mg of plasmalogen, 3.0 mg of sodium metabisulfite, 0.8 mg of methylparaben, 0.1 mg of propylparaben, and an appropriate amount of sterilized distilled water for injection were prepared by using a general method to have the total volume of 2 ml, and the resultant was filled in an 2 ml ample and sterilized to prepare an injection.

### <Formulation Example 2> Preparation of tablet

10 mg of batyl alcohol, 100 mg of lactose, 100 mg of starch, and an appropriate amount of magnesium stearic acid were mixed, and molded into a table by using a common tablet preparation method.

### <Formulation Example 3> Preparation of capsule

10 mg of batyl alcohol, 50 mg of lactose, 50 mg of starch, 2 mg of talc, and an appropriate amount of magnesium stearic acid were mixed, and filled in a gelatin capsule by using a common capsule preparation method to prepare a capsule.

Hereinafter, preparation examples of health food by using the plasmalogen or an analog thereof according to the present invention will be described, but the examples are for the purpose of illustrations only, not intended to limit the scope of the invention.

### <Preparation Example 1> Preparation of health food

10 mg of batyl alcohol, an appropriate amount of vitamin mixture (70 µg of vitamin A acetate, 1.0 mg of vitamin E, 0.13 mg of vitamin B1, 0.15 mg of vitamin B2, 0.5 mg of vitamin B6, 0.2 µg of vitamin B12, 10 mg of vitamin C, 10 µg of biotin, 1.7 mg of nicotinic acid amide, 50 µg of folic acid, and 0.5 mg of calcium pantothenate), and an appropriate amount of inorganic mixture (1.75 mg of iron (II) sulfate, 0.82 mg of zinc oxide, 25.3 mg of magnesium carbonate, 15 mg of potassium phosphate, 55 mg of calcium phosphate, 90 mg of potassium citric acid, 100 mg of calcium carbonate, and 24.8 mg of magnesium chloride) were mixed, granules were prepared, and health food was prepared by using a common method.

## Claims

1. A pharmaceutical composition for use in preventing or treating nonalcoholic steatohepatitis, the pharmaceutical composition comprising a batyl alcohol as an effective component.

2. The pharmaceutical composition for use of claim 1, wherein the pharmaceutical composition decreases accumulation of neutral fats, suppresses expression of inflammatory cytokine genes, and increases fatty acid oxidation of peroxisome.

3. The pharmaceutical composition for use of claim 1, wherein the batyl alcohol is contained at an amount in a range of about 0.01 part to about 90 parts by weight based on 100 parts by weight of the total weight of the pharmaceutical composition.

4. A health food composition for use in relieving or preventing non-alcoholic steatohepatitis, the health food composition comprising a batyl alcohol as an effective component.

## Patentansprüche

1. Pharmazeutische Zusammensetzung zur Verwendung bei der Prävention oder Behandlung nicht-alkoholischer Steatohepatitis, wobei die pharmazeutische Zusammensetzung einen Batylalkohol als eine wirksame Komponente umfasst.

2. Pharmazeutische Zusammensetzung zur Verwendung nach Anspruch 1, wobei die pharmazeutische Zusammensetzung die Akkumulation neutraler Fette verringert, die Expression inflammatorischer Cytokingene unterdrückt und die Fettsäureoxidation von Peroxisom erhöht.

3. Pharmazeutische Zusammensetzung zur Verwendung nach Anspruch 1, wobei der Batylalkohol in einer Menge in einem Bereich von ungefähr 0,01 Gewichtsteilen bis ungefähr 90 Gewichtsteilen, bezogen auf 100 Gewichtsteile des Gesamtgewichts der pharmazeutischen Zusammensetzung, enthalten ist.

4. Reformkostzusammensetzung zur Verwendung bei der Linderung oder Prävention nicht-alkoholischer Steatohepatitis, wobei die Reformkostzusammensetzung einen Batylalkohol als eine wirksame Komponente umfasst.

## Revendications

1. Composition pharmaceutique pour son utilisation dans la prévention ou le traitement de la stéatose hépatique non alcoolique, la composition pharmaceutique comprenant un alcool batylique en tant que composant efficace.

2. Composition pharmaceutique pour son utilisation selon la revendication 1, dans laquelle la composition pharmaceutique réduit l'accumulation de graisses neutres supprime l'expression de gènes des cytokines inflammatoires, et augmente l'oxydation des acides gras dans le péroxysome.

3. Composition pharmaceutique pour son utilisation selon la revendication 1, dans laquelle l'alcool batylique est contenu en une quantité dans une plage d'environ 0,01 partie à environ 90 parties en poids sur la base de 100 parties en poids du poids total de la composition pharmaceutique.

4. Composition d'aliments naturels pour son utilisation dans le soulagement ou la prévention de la stéatose hépatique non alcoolique, la composition d'aliments naturels comprenant un alcool batylique en tant que composant efficace.
